# EUROPEAN PATENT SPECIFICATION

(11) **EP 1 265 642 B1**
(45) Date of publication and mention of the grant of the patent: **27.12.2006**
(21) Application number: 01910698.8
(22) Date of filing: 14.02.2001
(51) Int. Cl.: A61L 2/26, A61L 2/14, A61L 2/07, A61L 2/20

(54) **FILTERED GAS PLASMA STERILIZATION CONTAINER WITH IMPROVED CIRCULATION**
STERILISIERBEHÄLTER MIT GEFILTERTEM GASPLASMA UND VERBESSERTER ZIRKULATION
RECIPIENT DE STERILISATION A PLASMA GAZEUX FILTRE AVEC CIRCULATION AMELIOREE

(30) Priority: 23.02.2000 US 184299 P
(43) Date of publication of application: 18.12.2002
(73) Proprietor: Case Medical, Inc., Ridgefield, NJ 07657 (US)
(72) Inventor: FRIEZE, Allan, S., Alpine, NJ 07620-0472 (US); FRIEZE, Marcia, A., Alpine, NJ 07620-0472 (US)
(74) Representative: Lenzing, Andreas
(86) International application number: PCT/US2001/004786
(87) International publication number: WO 2001/062302

(56) References cited:
- AU-B- 202 334
- US-A- 4 716 025
- US-A- 4 783 321
- US-A- 4 915 913
- US-A- 5 324 489
- US-A- 5 372 787
- US-A- 5 394 983
- US-A- 5 524 755
- US-A- 5 628 970
- US-A- 5 650 693
- US-A- 5 732 821
- US-A- 5 741 460

## Description

### BACKGROUND OF THE INVENTION

### 1. Field of the Invention

The invention relates, in general, to a method for sterilizing medical instruments, in particular, to a sterilization container useful for flash sterilization and gas plasma sterilization, which includes a filter that permits maximum gas sterilant penetration and prevents microorganisms and dust from entering, and which permits for stat flash sterilization by restricting the volume, and therefore the mass, of instruments placed into the container by utilizing a basket which fits into the container.

### 2. Description of Related Art

Steam sterilization is a common method used for the sterilization of items, especially medical instruments by processing the items in an autoclave and exposing them to high-pressure steam. This method requires the wrapping of individual items, heating the items with steam and then waiting for a drying/cooling period. Often during surgical procedures commonly used instruments are needed on a "stat" basis, i.e., the instruments need to be quickly sterilized after use or inadvertent contamination. Under such circumstances the standard autoclave method would take too long. An alternative sterilization method, which can be used under these circumstances, is known as flash sterilization. In flash sterilization metal instruments are not wrapped but are heated directly by the steam allowing sterilization in a reduced period of time. One drawback to the use of flash sterilization is the lack of a drying period. When the items are still moist and hot from sterilization, microorganisms and dust can contaminate the items when they are transported from the autoclave/sterilizer. Nevertheless, flash sterilization results in reduced exposure time.

Sterilization time can also be reduced by limiting the amount of instruments placed into the sterilization container. If too many instruments with too much mass are placed into the sterilization container, "stat" sterilization will not be effective. One method to restrict the amount of instruments placed into the sterilization container is to weigh the instruments. Weighing of instruments, however, can be time consuming in itself and difficult to control given the various sizes, shapes and odd surface areas of medical instruments.

One common design for containers for flash sterilization is described in U.S. Patents 5,097,865 and 4,748,003. Such containers use valves which require greater than atmospheric pressures to open the valves and allow the high-pressure steam to enter the container but are closed under normal pressure conditions. This approach has a number of disadvantages. Such containers must be opened to allow the steam to escape, thus breaking the sterile field. Even if kept sealed, these containers cannot maintain the sterile field for longer than 24 hours. Also, the high temperature, high-pressure valves needed for this method are very complex and very expensive. In addition, such containers do not provide an indication as to whether or not the valve properly functioned to allow the high pressure steam to enter the container.

In addition to flash steam sterilization the industry is beginning to use gas plasma as an alternative. One commercially available gas plasma system is sold as STERRAD^{®} by Advanced Sterilization Products, a division of the Johnson & Johnson Company. Gas plasma has known advantages over steam sterilization, including sterilizing at a lower temperature than required for steam sterilization, which is beneficial when sterilizing temperature-sensitive devices. However, it has been learned that frequently the sterilizing gas plasma does not reach all important surfaces on the inside of the sterilization container, especially where long tubular instruments or cables are contained. Accordingly, there are believed to be very few sterilization containers approved for use with gas plasma, especially in the mid-size range. Clearly a technique is missing in the prior art to guarantee satisfactory circulation of gas plasma within a sterilization container, especially where it is critical to reach the edges and corners of the interior of the sterilization container and to penetrate internal components such as laparascopic guides and tubing. The present invention, however, maintains its efficacy when utilized with gas plasma as the sterilant.

The US-A 491 591 3 discloses a sterilisation container comprising a lid and bottom part, both parts are anodised aluminium and each part comprising a set of holes covered by a filter material permeable to a sterilising gas. The anodised body and cover members are hard coated to provide a scratch-resistant and light weight construction.

### SUMMARY OF THE INVENTION

The invention is defined according to the claims.

Briefly described, the invention comprises a sterilization for sterilizing items, which allow for extended, sterile storage of the sterilized items. The flash sterilization method uses a sterilization container having a pan, a cover and one or more filters for preventing dust and microorganisms from entering the container and contaminating the sterilized items while still allowing gas plasma in and out of the container during the sterilization process. These containers can be used in the flash sterilization process commonly used in surgical theaters. The filter can be permanently mounted in the container or can be removable for replacement with new or different types of filters. Removable filters will allow for the retrofitting of currently used containers with the filters so that new containers do not need to be purchased to take advantage of the filtered flash sterilization method of the present invention. The filter can be removably attached to the container, manufactured as an integral part of the container, or incorporated into a self-contained removable filter unit.

A filter is attached to the sterilization container through a filter retainer. The filter retainer has a plurality of gas plasma penetration holes which can be of various sizes and shapes allowing sufficient gas plasma to enter the container. The filter retainer also comprises one or more gaskets for maintaining a seal between the filter retainer and the sterilization container as well as a locking means for removably attaching the retainer to the container.

Another aspect of the present invention allows for instruments to be sterilized on a "stat" basis by utilizing a basket which limits the amount of instruments that can be placed into the container for sterilization. When instruments need to be sterilized on a "stat" basis, for example, because the instrument became contaminated during the surgical procedure and no other such instrument is available, the time required to sterilize the instrument can be drastically reduced by limiting the amount or volume of instruments sterilized through utilization of a size restricted basket containing the instruments which would fit into the sterilization container. This indirectly limits the total mass of instruments to be sterilized, which in turn, reduces the sterilization time.

Another alternative embodiment of the invention provides for a single set of vent holes in the center of the lid, or cover, of the container and two sets of vent holes, arranged in a circular fashion, located in the base of the pan or bottom of the container. The second and third sets of vent holes in the base are located on opposite sides of the minor axis center line of the base in such a way that they do not overlap. Gas plasma passing through the first set of vent holes in the lid is then forced to travel to the extremes of the container in order to be exhausted thereby guaranteeing that all parts of the tray or sterilizable instruments on the inside come into contact with the gas plasma as well as the edges and corners and interior of the container.

According to yet another embodiment of the invention, a pair of vent means, comprising a first and fourth set, are located in the lid in a manner similar to the way the second and third set of vent holes are located in the base. This also helps to guarantee thorough circulation of the gas plasma within the container. These two improvements are especially suited for use with mid-size sterilizable containers that employ gas plasma as the sterilizing agent. This invention, however, enhances the efficacy of gas plasma sterilization. The first, second, third and fourth sets of vent holes are preferably each arranged in four concentric circles having the holes on their circumference. Other alternative, symmetrical patterns, like square, would also be acceptable. The keeper plate on the bottom of the container preferably includes a similar set of holes, but offset so that there can be no "strikethrough" of sharp objects through the filter underneath the series of vent holes but above the keeper plate.

These and other features of the invention may be more fully understood by reference to the following drawings.

### BRIEF DESCRIPTION OF THE DRAWINGS

Fig. 1A is a perspective view of the flash sterilization container invention.
Fig. 1B is a top perspective view of the flash sterilization container cover with a filter retainer.
Fig. 1C is a perspective view of the flash sterilization container invention with the top surface of the lid having a D-ring attached to it.
Fig. 2A is an exploded perspective view of the flash sterilization container filter invention.
Fig. 2B is a partial view of a cover opening configuration.
Fig. 3A is a side elevational view of the filter retainer invention.
Fig. 3B is a top plan view of the filter retainer invention with the locking means in the locked position.
Fig. 3C is a top plan view of the filter retainer invention with the locking means in the unlocked position.
Fig. 4A is a partial, side elevational view of a flash sterilization container cover with an incorporated filter.
Fig. 4B is a partial, side elevational view of a flash sterilization container and a filter cartridge.
Fig. 5A is an exploded view of an alternative embodiment of the invention especially suitable for use with gas plasma in which the base of the container includes two sets of circular vent holes located on opposite sides of the center line of the minor axis of the container.
Fig. 5B is a top plan view of the lid of the alternative embodiment illustrated in Fig. 5A.
Fig. 5C is an elevational cross-sectional view of the lid illustrated in Fig. 5B.
Fig. 5D is a partial, cross-sectional exploded view of the top, or first set, of circular vent means illustrated in a manner in which the pull ring is attached to the lid.
Fig. 5E is a detailed, cross-sectional end view of the lid of Fig. 5B illustrated in a manner in which the snap-on post attaches to the filter retainer plate.
Fig. 5F illustrates the bottom, or pan, of the gas plasma alternative embodiment illustrating how the second and third set of circular vent means are located on opposite sides of the center line of the minor axis of the bottom portion.
Fig. 6A is an exploded view of another alternative embodiment of the gas plasma container illustrating a fourth set of circular vent means located adjacent to said first set of circular vent means in the top of lid of the container.
Fig. 6B is a top plan view of the lid of the alternative embodiment illustrated in Fig. 6A.
Fig. 6C is a side elevational cross-sectional view of the lid illustrated in Fig. 6B.
Fig. 6D is a partial, detail exploded view of the first set of circular vent means illustrating the manner in which the pull ring is attached to the lid.
Fig. 7A is a perspective view of the preferred embodiment of the instrument limiting basket with a divider system.
Fig. 7B is a side, elevation view of the basket with handles and feet.
Fig. 7C is top plan view of the base of the basket with the divider system removed.
Fig. 7D is a side, elevation view of the basket handle and feet shown in Fig. 7A.

### DETAILED DESCRIPTION OF THE INVENTION

During the course of this description like numbers will be used to identify like elements according to the different figures that illustrate the invention.

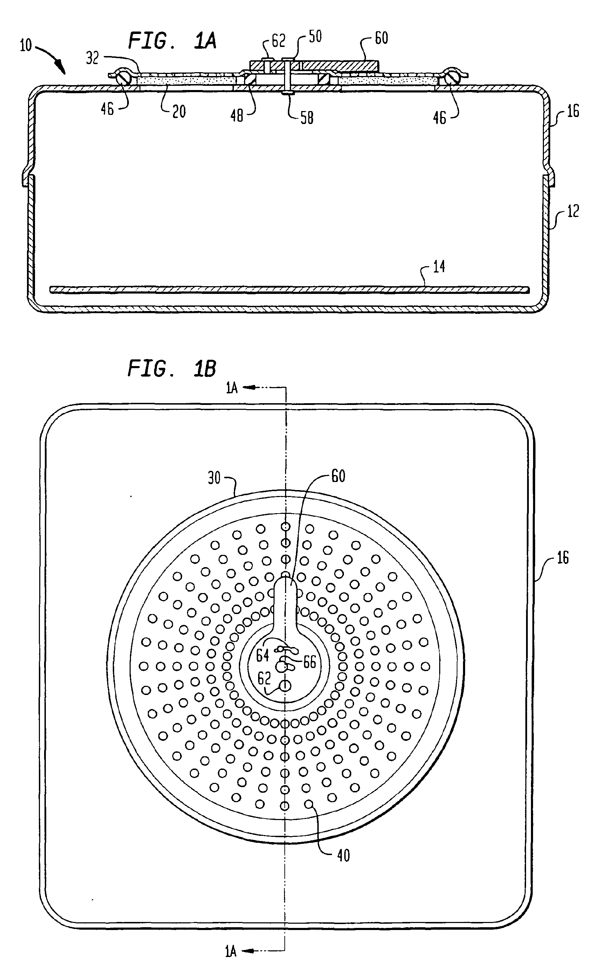

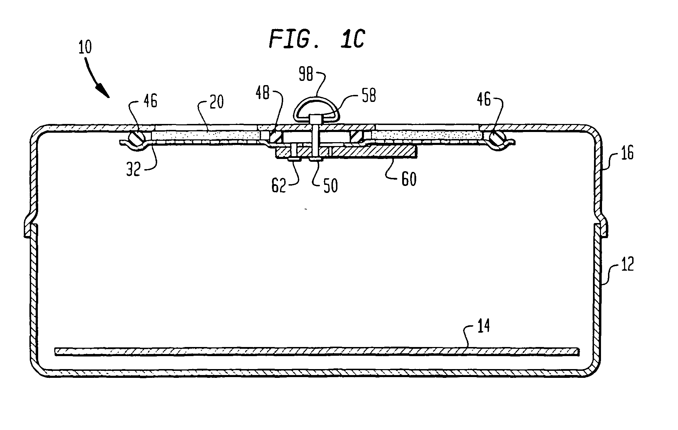

A flash sterilization container **10** is illustrated in Fig. 1. A Sterilization container **10** comprises a pan **12,** which forms the bottom of the container that holds and supports a conventional sterilizable tray **14,** and a cover **16,** which forms the top of the container. The cover is removably attached to the pan to form a hermetically sealed container. This can be accomplished by conventional means such as hinges and clamps and a sealing gasket. The cover **16** is provided with an opening **18** at its top. This opening **18** is covered with a filter **20** to allow gas plasma to enter and exit the container through the opening by passing through the filter. The filter can be removably or permanently attached to the cover. This filter is made of a material, or combination of materials, such that the filter is permeable to the flow of gas plasma but will inhibit dust or other airborne particles or microorganisms from passing through. Examples of such materials include paper, TEFLON^{®}, a registered trademark of E.I. Du Pond de Nemours and Co., Inc., porous stainless steel, polysulfone, and hydrophobic material, such as GOR-TEX^{®}, a registered trademark of W.L. Gore & Associates, and Kimguard^{®}, a trademark of the Kimberly-Clark Corporation. The filter is attached to the cover by means which will prevent any gas plasma, dust or other airborne particles or microorganisms from passing through the opening in the cover without passing through the filter.

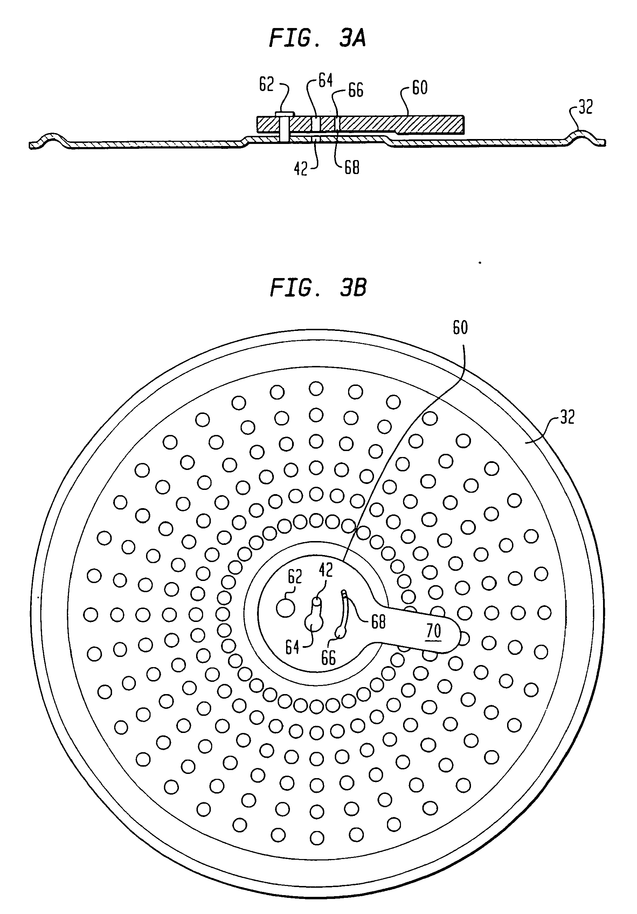

The filter **20** is placed over the opening **18** in the top of the cover **16** and the filter is attached to the cover by a filter retainer **30.** One embodiment of such a filter retainer is illustrated in figures 1A, 1B, 2A, 3A and 3B. The filter retainer 30 comprises a filter retainer disc **32** and a means for sealing the filter retainer disc to the cover. The filter retainer disc has an inner disc **34,** a middle ring **36,** and an outer ring **38.** The middle ring **36** has a plurality of holes **40** to allow the flow of gas plasma through the filter retainer disc **32,** through the filter **20,** and through the opening **18** in the cover **16.** The filter retainer can have one or more sealing means for forming a seal between the filter and the cover. The outer ring **38** has a means for forming a seal between the filter and the cover. In one embodiment the outer ring has an inverted-U shaped cross-section. A gasket **46** is placed in the inverted-U outer ring and can be made of silicone, neoprene, TEFLON^{®}, a registered trademark of E.I. Du Pond de Nemours and Co., Inc., or any other suitable material. Inner disc **34** may also have a sealing means if necessary, such as a gasket **48.**

The preferred metal that is used for the container is the aluminum alloy 6061 with a T-6 heat treatment. This alloy (6061 T-6) is treated electrolytically in a bath containing sulfuric acid to produce a uniform anodic coating on the metal surface. The thickness of the aluminum anodizing should not exceed 0.5 mils (.0005 inches) and preferably should have a coating between 0.2 mils (.0002 inches) and 0.3 mils (.0003 inches). This is less than the normally allowed specifications which allows up to 0.7 mils (.0007 inches). Coating below 0.5 mils results in improved sterilization characteristics in the container over thicker coatings and it appears that the coatings in the range of 0.2 mils to 0.3 mils provides the best results.

All parts of the container should be anodized after all beat treatment, machining, welding, forming and perforating has been completed. The anodic coating should not be applied to assemblies which will entrap the electrolyte in joints or recesses. Assemblies, which contain non-aluminum parts such as steel, brass, or organic substances which would be attacked by pretreatment or anodizing solutions or prevent uniform formation of the anodic or prevent uniform formation of the anodic coating, shall not be anodized as an assembly unless proper protection can be provided to ensure the anodic coating meets the specification, e.g., masking of non-aluminum surfaces or electrically insulating such surfaces. It is important to keep the aluminum parts clean of all foreign substances, such as grease and welding flux and free of metals, such as steel or iron, which would accelerate corrosion of the aluminum alloy. After applying a uniform anodic coating, all parts should be completely sealed in a known manner.

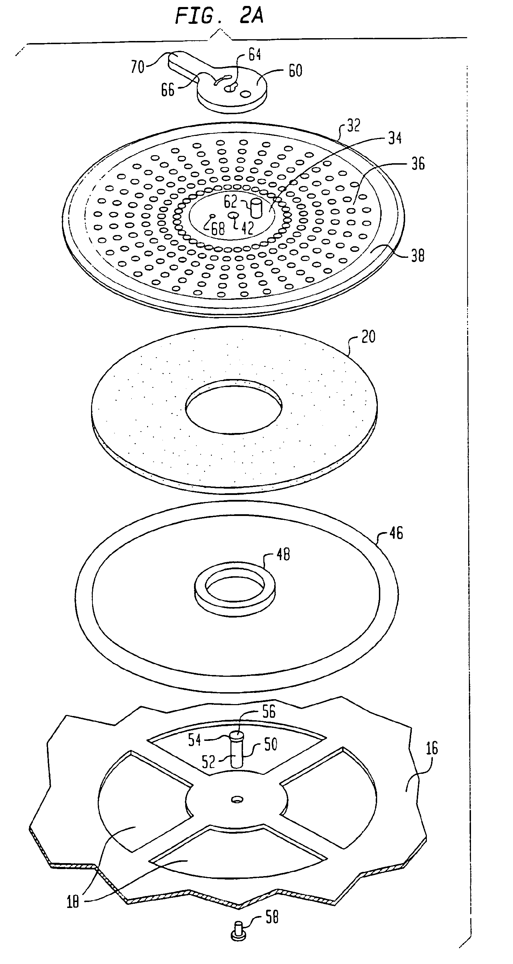

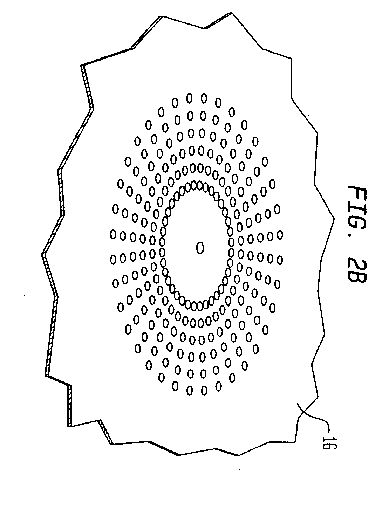

A means for attaching the filter retainer to the cover is illustrated in figures 1A, 1B, 2A, 3A, and 3B. Pin **50** extends upwards from the top surface of the cover **16.** Inner disc **34** of the filter retainer disc **32** has a hole **42** for accepting pin **50.** Pin **50** has sufficient length to extend past the top surface of the filter retainer **30** when the filter retainer is placed on the cover **16** allowing the hole in the inner disc **42** to engage the pin **50**. Pin **50** is preferably located in the center of the opening **18** in the cover **16.** To locate the pin in the proper position, the opening in the cover can be, for example, a circular opening with cross pieces such as those illustrated in figure 2A. In an alternate embodiment, the opening in the cover can be comprised of a multiplicity of smaller openings in the cover, as depicted in Fig. 2B. A locking means is located on the inner disc which engages the pin **50** and holds the filter retainer in place.

A preferred locking means is comprised of a sliding plate **60** which is movably attached to the inner disc **34** of the filter retainer disc **32** with a hinge pin **62** extending from the inner disc **34** of the filter retainer disc **32,** so that the sliding plate **60** rotates about the center of the hinge pin **62** while remaining in contact with the inner disc. The sliding plate **60** has an arc-shaped pin slot **64** having an effective radius equal to the distance from the hinge pin **62** to the hole in the center of the inner disc **42,** whereby the center of the hole in the inner disc maintains alignment with the center of the arc-shaped slot throughout the rotation of the sliding plate.

The retainer pin **50** is comprised of a cylindrical body **52** having opposite ends and an outside diameter essentially equal to the inside diameter of the hole **42** in the filter retainer disc, a cylindrical neck **54** having a diameter smaller than the diameter of the body and equal to the width of the arc shaped slot **64,** and a cylindrical head **56** having a top and a bottom and having a diameter larger than the pin neck **54,** preferably equal to the diameter of the pin body **52.** One end of the pin body **52** is attached to the cover **16** by conventional means, such as, a rivet **58,** a screw, a thread, or a spot weld. Pin neck **54** is attached to the end of the pin body **52** opposite the attachment to the cover. The bottom of the pin head is attached to the pin neck at the end opposite the pin neck's attachment to the pin body. The retainer pin can be made of separate elements attached by conventional means or preferably manufactured from a single piece of stock. The length of the pin body is essentially equal to the distance from the top surface of the cover to the top surface of the inner disc. The length of the pin neck is at least equal to the thickness of the sliding plate. The combined length of the pin body and pin neck is such that the bottom of the pin head is slightly lower than the top surface of the sliding plate **60** so that when the arc-shaped pin slot **64** engages the pin neck **54,** the filter retainer disc **32** will be forced toward the cover, compressing the gaskets **46, 48,** and creating a seal between the filter retainer **30** and the cover **16.** The top of the pin head **56** can have a taper to facilitate the insertion of the retainer pin **50** through the hole in the filter retainer disc **42** and arc-shaped slot in the sliding plate **64.**

The arc shaped slot **64** has a width essentially equal to the outside diameter of the pin neck and a length at least twice as long as the outside diameter of the pin head. At one end of the arc-shaped slot **64,** the width of the slot is increased to allow the pin head to pass through the arc-shaped slot.

A filter is attached to the cover with the filter retainer by placing a filter **20** over the hole **18** in the cover **16,** the sliding plate **60** is positioned so that the end of the arc-shaped slot **64** having an increased width is aligned with the hole in the inner disc **42,** the filter retainer **30** is then placed over the opening in the cover **16** so that the retainer pin **50** passes through the hole in the inner disc **42** and the enlarged end of the arc-shaped slot **64,** and the sliding plate **60** is then rotated so that the arc-shaped slot **64** engages the pin neck **54,** thereby preventing the pin from passing back through the arc-shaped slot and thus attaching the filter retainer to the cover.

The filter retainer **30** has a means for limiting the rotation of the sliding plate **60** and facilitating the positioning of the sliding plate in an "open" position, where the enlarged end of the arc-shaped slot **64** lines up with the hole in the inner disc **42,** and a "locked" position, where the opposite end of the arc-shaped slot lines up with the hole in the inner disc. One embodiment of a limiting means incorporates an arc-shaped limiting slot **66** on the sliding plate **60.** The arc of the limiting slot 66 is parallel to the arc of the arc-shaped slot **64** and has an effective radius larger than the radius of the arc-shaped slot **64.** A locating pin **68** is attached to, and extends from, the inner disc **34** such that it engages one end of the limiting slot **66** when the sliding plate **60** is in the locked position and engages the opposite end of the arc-shaped slot when the sliding plate is in the unlocked position. The locating pin **68** is preferably hemispherical-shaped to facilitate the movement of the sliding plate **60** over the locating pin **68.** A hemispherical locating pin **68** can be made, for example, by inserting and attaching a ball bearing in a hole in the inner disc **34.** The width of the limiting slot **66** is slightly less than the diameter of the locating pin **68.** The width of the limiting slot **66** at each of the two, opposite ends, is enlarged slightly, forming two holes each having a diameter slightly larger than the diameter of the locating pin **68.** Consequently, the sliding plate **60** is held in the locked and open positions when the locating pin **68** engages each of the holes in the ends of the limiting slot **66,** requiring the application of an external force to move the sliding plate between the two positions.

The sliding plate **60** preferably has a handle **70** to facilitate moving the plate between the open and closed positions. The handle **70** preferably extends parallel to the plane of the sliding plate. The handle **70** can be attached to the sliding plate or manufactured with the sliding plate as a single piece.

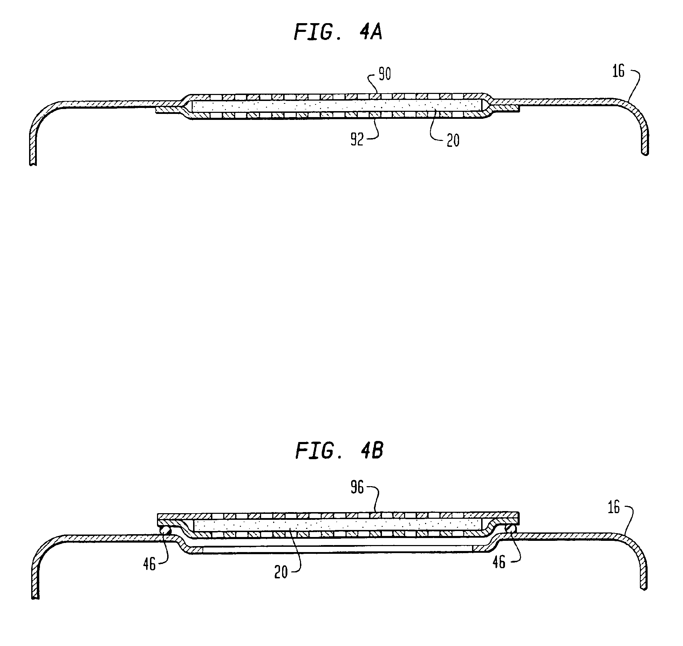

As described above, the sterilization container has a filter retainer mechanism. Alternative embodiments comprise having the filter manufactured as an integral part of the container as depicted in Fig. 4A, or having the filter incorporated into a self-contained removable filter unit or cartridge as depicted in Fig. 4B.

A further alternative embodiment comprises a D-ring attached to the end of pin **50** connected to the cover **16.** In this embodiment, the filter and filter retainer are mounted on the inside of the sterilization container. This arrangement permits the external D-ring to be used as a handle to lift the cover without coming into contact with the side edges of the cover **16,** thereby reducing the risk of contamination of the container contents.

While the foregoing embodiment works sufficiently well in a flash sterilization environment, it has been found that improvements to the basic structure of the invention are desirable if used with gas plasma. Gas plasma as a sterilization medium is fairly new and is available from, among others, Advanced Sterilization Products, a division of Johnson & Johnson, under the trademark STERRAD^{®}. Gas plasma has certain advantages over the prior art. For example, ethylene oxide has been banned thereby making it more difficult to find suitable alternatives. In addition, steam sterilization cannot be used with a number of modem tools, such as cannulas, lumens, scopes, fiber optic cables, and cameras, without damaging them. While gas plasma clearly has certain distinct advantages, it has been found that it does not operate suitably well with all types of containers that were suitable for use with steam sterilization. Part of the problem is that the gas plasma does not circulate as aggressively as steam inside the container, and does not reach areas such as the corners, thereby leaving the potential for unsterilized surgical instruments or the like. Steam sterilization avoids the absorption problem by having a super-saturated environment of water molecules. The present invention, however, has provided a way for making gas plasma acceptable for use in sterilization containers, especially those in the mid-size range. For the purposes of this disclosure, mid-size is defined as approximately 15 - 18 inches in length, 9 - 12 inches in width and 2 - 10 inches in depth. The container, especially in the mid-size container, has several advantages, including but not limited to, the following: 1) it cuts the exposure time in gas plasma sterilization; 2) it improves the effectiveness of gas plasma sterilization; and 3) it is more efficacious for gravity displacement applications.

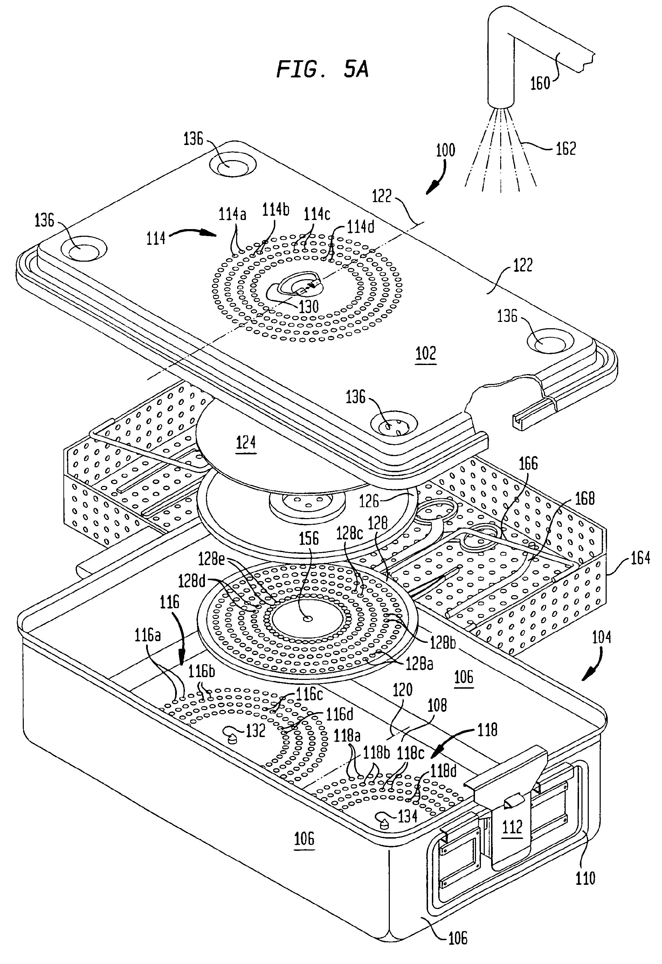

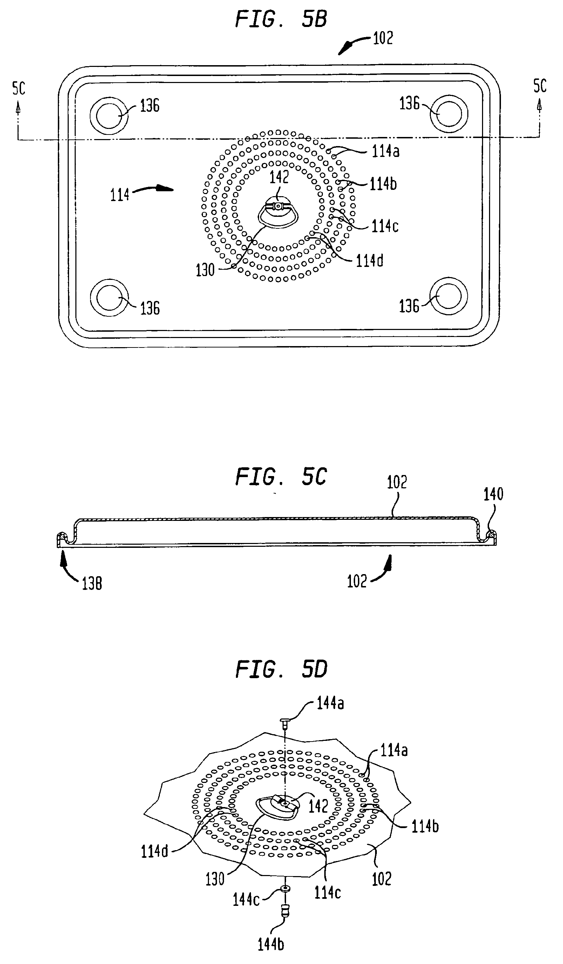

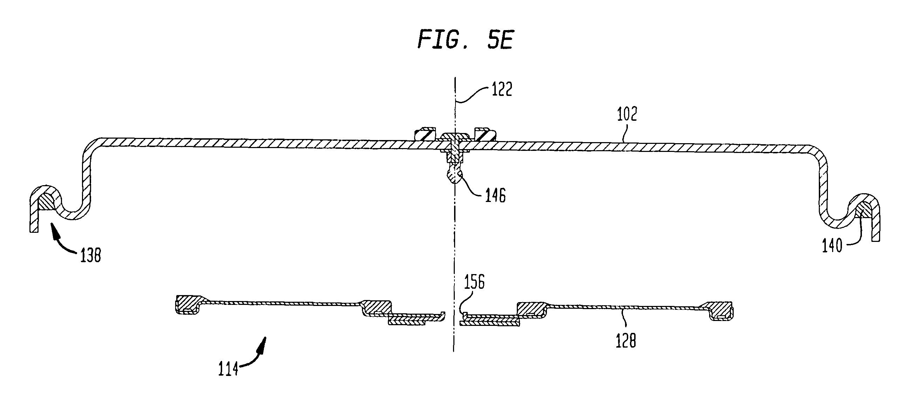

A first alternative embodiment **100** of the improved gas plasma sterilization container apparatus is illustrated in the exploded view of Fig. 5A. The container **100** includes a top or lid **102** that sits on top of a bottom or pan **104.** Bottom **104** includes four sidewalls **106** and a bottom or base **108.** A pair of wire handles or bales **110** are located on opposite ends of the bottom portion **104** and are held in place by a pair of lockable latches **112.**

The anodized surfaces on the lid **102** and pan **104** form an electrical insulation barrier between the two parts **102, 104.** Gas plasma is generally electrically charged and the lid **102** and the pan **104** appear to form, in their insulated assembly, a unique construction which appears to aid the gas plasma sterilization process within container **10.** The thinness of the anodized coating, i.e., less than 0.5 mils (0.0005 inches), further appears to improve the sterilization process with ultimate results appearing to be achieved when the anodized coating is in the range of 0.2 mils (0.0002 inches) to 0.3 mils (0.0003 inches). In particular, great results in achieving sterilization are achieved when the lid **102** and the bottom **104** are made out of 6061 T-6 aluminum.

A first set of vent holes **114** is located in top **102.** The vent holes **114** are preferably arranged as a group of four concentric circles with holes **114a, 114b, 114c and 114d** in each, respectively. In all, the total number of holes may range from 100 to 500 and have a size that ranges in diameter from, but not limited to, 3/16 inches to 5/16inches. The first set of vent holes **114** is located on the central axis **122** of the short dimension of the lid **102.** The first set of vent holes **114** allows the sterilizing medium **162** to pass into the container. A pull ring **130,** attached to a base **142** sits in the middle of the first set of vent holes **114** and is connected there by rivet assembly **144a, 144b, and 144c** as shown in exploded detail in Fig. 5D. The lid **102** also includes four recessed dimples **136** which are adapted to engage with complimentary dimples or projections in the base **108** (not shown) so that the containers **100** can be stacked on each other and permit circulation of gas plasma therethrough at the same time.

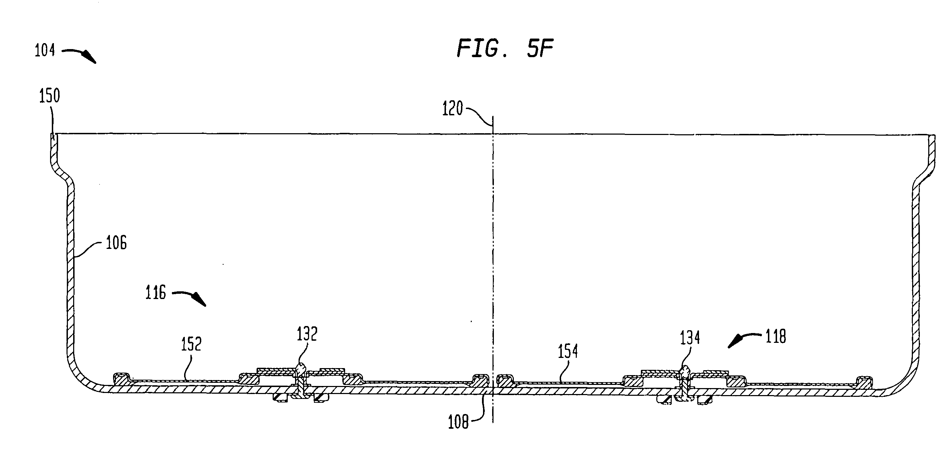

A second set of vent holes **116** and a third set of vent holes **118** are located in the base **108** on symmetrical opposite sides of center line **120** which represents the minor axis of the base **108.** The second set of vent holes **116** also comprises four concentric circles having holes **116a, 116b, 116c and 116d** of the same dimensions with regard to the first set of vent holes **114.** A hold-down stud **132** is located in the center of the concentric circles and is intended to make a snap fit with the retainer plate for the hydrophobic filter that goes therebetween. Similarly, the third set of vent holes **118** comprises four sets of concentric circles having holes **118a, 118b, 118c and 118d** therein. A central post or stud **134** is also located in the middle thereof and adapted to snap into and engage a hydrophobic filter retainer plate in the manner previously described with regard to the flash sterilization embodiment. Associated with the first set of vent holes **114** is a circular hydrophobic filter disk **124,** a hold down ring **126** and a perforated filter retainer plate **128.** A central hole **156** in the retainer plate **128** snaps into and engages a stud **146** in the container as illustrated in Fig. 5E. A similar set of hydrophobic filters, rings, and retainer plates is associated with the second and third set of vent holes **116** and **118** as illustrated in Fig. 5F. Hydrophobic filters **124** should be utilized when gas plasma acts as the sterilizing medium. The TYVEK^{®}, a trademark of E.I. du Pont de Nemours & Company, brand of polyethylene/polypropylene spun fiber is acceptable, as is Kimguard^{®}, a trademark of the Kimberly-Clark Corporation. The alternative embodiments **100 and 200** also work best with hydrophobic filters such as described above. In addition, hydrophobic filters do not absorb water, which allows for a quicker drying time. The concentric holes **128a, 128b, 128c, 128d** and **128e,** are preferably offset from the holes **114a, 114b, 114c, and 114d** so as to the prevent "strikethrough". That is to prevent sharp objects from entering the holes **114a, 114b, 114c and 114d** and exiting through **128a, 128b, 128c, 128d** or **128e.** As illustrated in Fig. 5C the top or lid **102** includes a groove **138** which retains a gasket **140** which sits on top of the upper lip **150** of the bottom or base pan **104** as shown in Fig. 5F. Fig. 5F also shows in further detail how the bottom perforated retainer plate **152** attaches to the bottom stud **132** and keeps a hydrophobic filter in place above the second set of vent holes **116.** Similarly, Fig. 5F also illustrates how another perforated filter retainer plate **154** engages snap on stud **134** to hold another hydrophobic filter in place above the third set of perforated vent holes **118.**

The structure just described works especially well with gravity displacement or gas plasma. The gas plasma enters through the vent holes **114,** passes through the hydrophobic filter **124** and emerges through the perforated base plate **128.** Because the top vent holes **114** are not located directly above the bottom pair of vent holes **116** and **118,** the gas plasma is forced to migrate, and become somewhat turbulent as it attempts to find an exit through the second and third set of circular vent holes **116** and **118,** respectively. This forces the gas plasma to more thoroughly mix and contact medical instruments or the like inside of the container **100** and also forces it further towards the corners and edges of the container. As a consequence, the invention described is one of a few containers, if any, that has been approved by major manufacturers for use with gas plasma. It is believed that no other sealed container in the mid-size range for sterilization of blades and cannulas has been approved at the present time. The present invention in the mid-size range with the offset sets of vent holes works in all methods of sterilization, including flash sterilization and gas plasma sterilization.

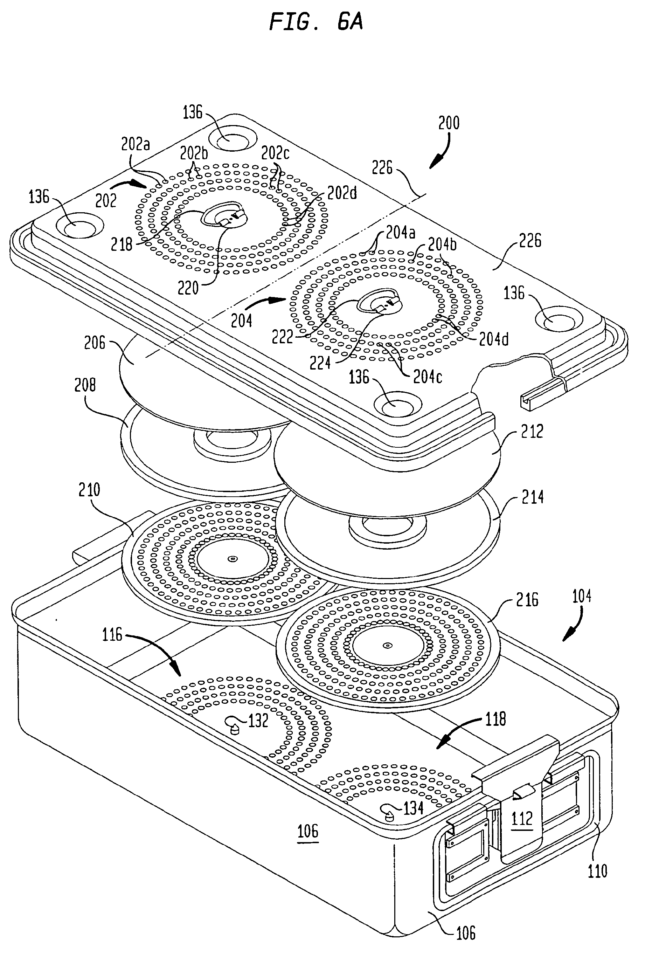

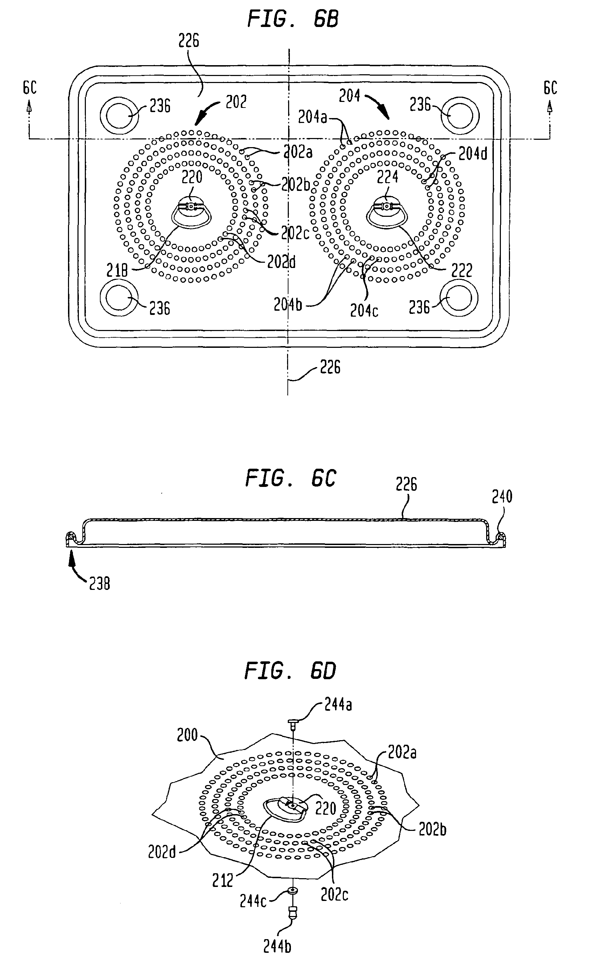

A second alternative embodiment **200** of the gas plasma version is illustrated in an exploded view shown in Fig. 6A. The base, or bottom pan **104** of the embodiment **200** is identical in all respects to the base **104** illustrated in Fig. 5A and associated with the first gas plasma alternative embodiment **100.** Namely, the base **104** also includes a pair of offset circular vent holes **116 and 118** each having a hydrophobic filter and a retainer plate associated therewith as seen, for example, in detail in Fig. 5F. The difference between embodiment **100 and 200** is that alternative embodiment **200** includes a pair of circular sets of vent holes **202 and 204** arranged symmetrically on opposite sides of the small dimension center line **226.** The structure of the first and second set of vent holes **202 and 204** is identical to the structure of the set of vent holes **114** in the lid **102** of embodiment **100** as illustrated in Figs. 5A - 5F. Namely, the first set of vent holes **202** comprises four concentric circles of vent holes **202a, 202b, 202c and 202d.** A pull ring **218** connected to a base **220** is located in the center of the concentric circles **202.** Pull ring **218** is attached to the base by a rivet assembly **244a, 244b,** and **244c** as illustrated in exploded detail view in Fig. 6D. Similarly, the second set of vent holes **204** comprises four concentric circles having vent holes **204a, 204b, 204c and 204d** arranged around a pull ring **222** attached to a base **224** and connected to the lid **226** in the same manner as illustrated in Fig. 6D. The first set of vent holes **202** has associated with it a hydrophobic filter disk **206,** a ring **208,** and a perforated retainer plate **210** that snaps and attaches to a post on the bottom side of the base plate **220** in the same manner that the post **146** of the embodiment **100** engages its perforated retainer plate **128** as illustrated in Fig. 5E. Similarly, another hydrophobic filter disk **212** is located under the second set of vent holes **204,** and has an associated ring **214** and perforated retainer plate **216** below it which also engages with a snap on post associated with pull ring **222** and base plate **224.** This second alternative embodiment **200** also provides for improved circulation of the gas plasma through the container so as to contact all the surgical instruments and the corners of the device.

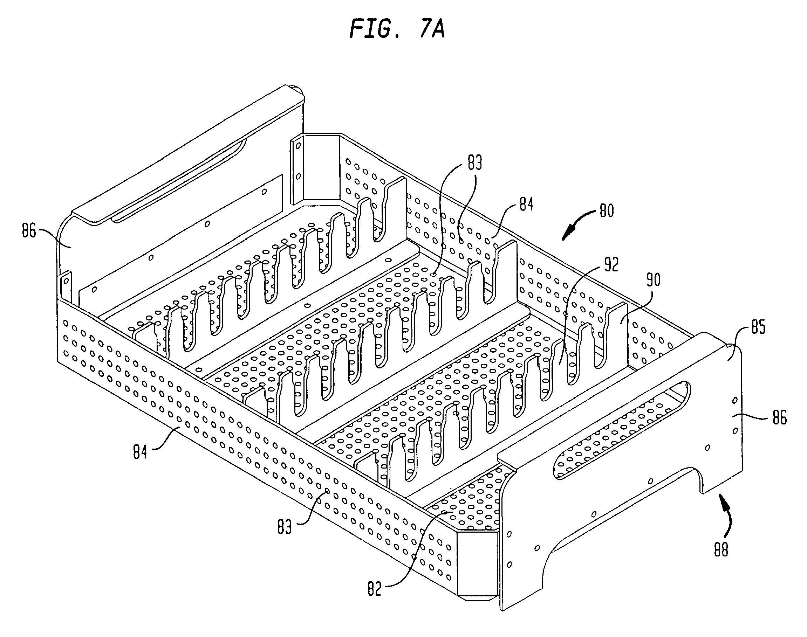

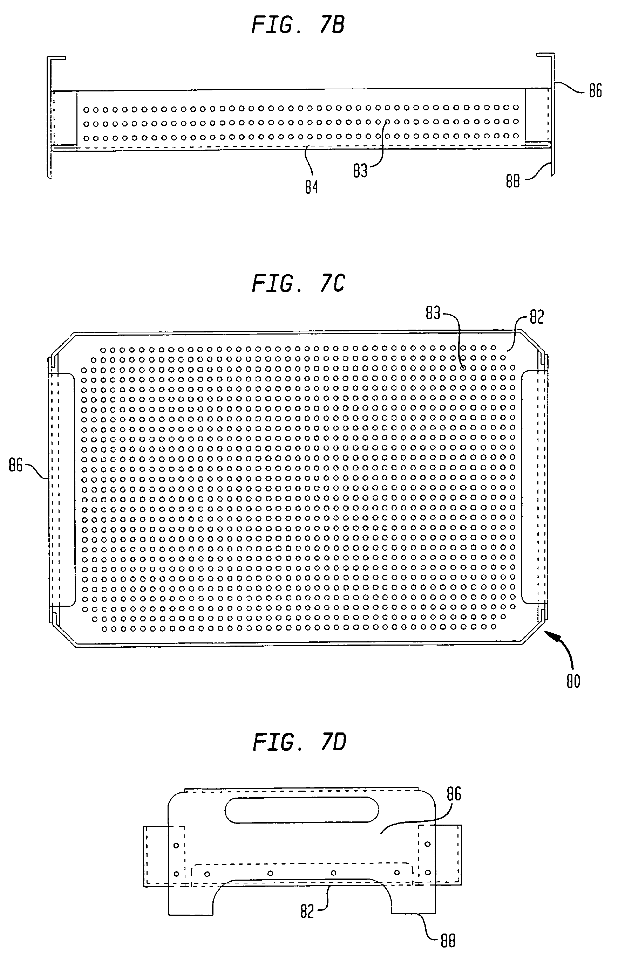

The present invention can be utilized for "stat" sterilization, for example, where immediate sterilization is required because an instrument becomes contaminated during surgery. Experimentation has shown that in order to reduce sterilization time, the amount, i.e., mass, of material to be sterilized must be limited. Limiting the amount of material to be sterilized can be accomplished by weighing the instruments. Weighing of instruments, however, is time consuming, difficult, and unreliable because the instruments are of varying masses and have odd surface areas. An improved method according to the present invention is to limit the amount of material for "stat" sterilization by utilizing a basket 80, as shown in Fig. 7A, which limits the amount of items sterilized by limiting the space in which instruments could be placed. This, in turn, limits the total mass of the instruments and guarantees that they can be sterilized sufficiently in a short period of time. Experimentation has shown that baskets which limit the volume of instruments to be sterilized to no more than twenty percent (20%) of the volume of the sterilization container allow for the most effective "stat" sterilization. In these experiments, a basket approximately 12 inches long, by 6 inches wide and 2 inches high, or a volume of approximately of 149 cubic inches, was utilized to determine the effectiveness of limiting the mass of instruments to be sterilized on a "stat" basis. The most effective use of the basket occurred in a sterilization container 18.1 inches long, by 11 inches wide and 4 inches high, or a volume of approximately 796 cubic inches.

The basket **80** of the preferred embodiment of the volume limiting present invention consists of a base **82,** and two side walls **84** attached to the base **82.** The base **82** and the side walls **84** are highly perforated **83.** The basket **80** also contains two handle fixtures **86** on opposite sides. The handle fixtures **86** contain feet **88** extending past the base **82,** and a handle portion **85** that extends beyond the side walls **84.** The feet **88** and handle extend **85** beyond the sidewalls **84** so that only one basket **80** could be placed in the container.

The basket **80** may also utilize a divider system **90** to limit the amount of instruments placed in the basket **80.** The divider system **90** consists of a series of brackets **92** which could be scalloped to prevent instruments from being tightly packed.

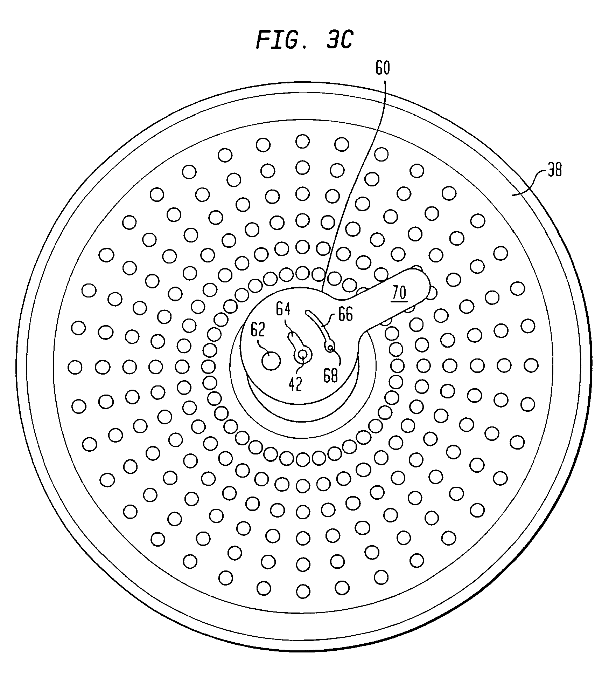

In summary, the gas plasma embodiments make it possible to convert a flash sterilization container such as illustrated in Figs. 1A through 4B into a version which is imminently acceptable and suitable for use with mid-size sterilization containers. Not only does it work with mid-size containers, it also permits sterilization to take place in approximately half the time and works especially well in a gravity displacement environment. Moreover, delicate instruments, such as cameras which cannot be sterilized with steam or ethylene oxide, can be effectively sterilized.

It is believed that by making the base and lid of the container out of aluminum and anodizing the aluminum to a thickness of 0.5 mils (0.0005 inches) or less that improved sterilization results are achieved in a gas plasma sterilization medium. These results, it is believed, allowed sterilization containers that are so constructed to pass strict approval requirements. It is further believed that by maintaining the anodized coating in the 0.2 mils (0.0002 inches) to 0.3 mils (0.0003 inches) range, optimum sterilization results are achieved. The use of 6061 T-6 aluminum is further believed to add to these improved sterilization results. It is additionally believed that by electrically insulating the top of the container from the bottom of the container improves sterilization results with a gas plasma sterilization medium occur. This electrical insulation can be achieved by anodizing the mating parts (top and bottom) of the container as indicated and preventing other paths of electrical current from flowing between the two container parts, e.g., clamps holding the two parts together without appropriate electrical insulation.

## Claims

1. A metal sterilization container (100,200) for sterilizing items in a gas plasma sterilization medium, the container (100,200) comprising:
an aluminium lid (102, 226) having a first set of vent holes (114, 202; 204);
an aluminium bottom (104) having a second set of vent holes (116), the aluminium bottom (104) attachable to the aluminium lid (102, 226);
a first and second filter medium (124, 206, 212), permeable to the flow of gas plasma, respectively associated with the first and second set of vent holes (114, 11G; 202, 204)
**characterised in that**
an anodic coating substantially applied to the aluminium lid (102, 226) and the aluminium bottom (104), the anodic coating having a thickness substantially not exceeding 0.5 mils (0.0005 inches; 12.7 µm).

2. The metal sterilization container of claim 1, wherein the aluminium comprises 6061 T6.

3. The metal sterilization container of claim 1, wherein the anodic coating comprises a thickness substantially not exceeding 0.3 mils (0.0003 inches; 7.62 µm).

4. The metal sterilization container of claim 1, wherein the anodic coating comprises a thickness substantially below 0.2 mils (0.0002 inches; 5.08 µm).

5. The metal sterilization container of claim 1, wherein the anodic coating comprises a thickness substantially between 0.2 mils (0.0002 inches; 5.08 µm) and 0.3 mils (0.0003 inches; 7.62 µm).

6. The metal sterilization container of claim 1, wherein the anodic coating comprises a thickness substantially between 0.2 mils (0.0002 inches; 5.08 µm) and 0.5 mils (0.0005 inches; 12.7 µm).

7. The metal sterilization container of claim 1, wherein the anodic coating comprises a thickness substantially between 0.3 mils (0.0003 inches; 7.62 µm) and 0.5 mils (0.0005 inches; 12.7 µm).

8. The metal sterilization container of claim 1, wherein the first set of vent holes and the second set of vent holes (114, 116; 202, 204) are offset from each other.

9. The metal sterilization container of claim 1, further comprising a gasket attachable between the aluminium lid and the aluminium bottom.

10. The metal sterilization container of claim 1, further comprising a filter retainer plate (32) attachable to the aluminium lid (16), the filter retainer plate (32) having a set of vent holes substantially offset relative to the first set of vent holes (114).

11. The metal sterilization container of claim 1, wherein the first retainer plate (32. 128) and the aluminium lid (16, 102) define a gap in the region of the vent holes (114) in which the first filter medium (20, 124) is placed.

12. The metal sterilization container of claim 1, wherein the aluminium bottom (108) and the second retention plate (154) define a gap in the region of the second set of vent holes in which the second filter medium is placed.

13. The metal sterilization container of claim 1, further comprising a gasket (46,48) attached to said first retainer plate (32) positioned to create a seal with said lid when in a sealing position.

14. The metal sterilization container of claim 1, comprising one or more gaskets (46, 48)

15. The metal sterilization container of claim 13, wherein the gasket (46, 48) can be made of silicone, neoprene or Teflon®.

16. The metal sterilization container of claim 1, further comprising a gasket attached to said second retainer plate positioned to create a seal with said bottom when in a scaling position.

17. The metal sterilization container of claim 1, wherein the filter medium is made of paper. Teflon, porous stainless steel, polysulfone, hydrophobic material, and mixtures thereof.

18. The metal sterilization container of claim 1, further comprising a third set of vent holes substantially located in the aluminium bottom.

19. The metal sterilization container of claim 1, wherein the aluminium lid and the aluminium bottom are substantially electrically insulated from each other.

20. A system for sterilizing items in a container with a gas plasma, the system comprising:
means for introducing gas plasma and the container of claim 1;

21. A method for sterilizing instruments in a metal container having a lid and a bottom, the lid having a first set of vent holes and a first filter medium, the bottom having a second set of vent holes and a second filter medium, the method comprising the steps of:
placing at least one instrument in the metal container;
passing a gas plasma sterilization medium though the first set of vent holes and the first filter medium;
causing the gas plasma sterilization medium to turbulently flow within the metal container;
substantially restricting the electrical current between the lid and the bottom, wherein the lid and bottom comprise an anodization layer with a thickness not greater than 0.5 mils (0.0005 inches; 12.7 µm); and
passing the gas plasma sterilization medium through the second set of vent holes and the second filter medium.

22. A method of manufacturing an aluminium sterilization container having a lid and a bottom, the method comprising the steps of:
forming a fist set of vent holes in the lid;
forming a second set of vent holes in the bottom; and
applying a substantially uniform anodic coating to the lid and the bottom, the anodic coating having a thickness no greater than 0.5 mils (0.0005 inches; 12.7 µm).

23. The metal sterilization container of claim 1, further comprising a second filter retainer plate attachable to the aluminium bottom, the filter retainer plate having a set of vent holes substantially offset relative to the second set of vent holes.

24. The metal sterilization container of claim 16, wherein said hydrophobic material is a polyethylene/polypropylene.

## Patentansprüche

1. Metallischer Sterilisationsbehälter (100,200) für die Sterilisation von Gegenständen in einem Gasplasmasterilisationsmedium, wobei der Behälter (100,200) umfasst:
einen Aluminiumverschluss (102,226) mit einem ersten Satz von Lüftungslöchern (114,202;204);
einen Aluminiumboden (104) mit einem zweiten Satz von Lüftungslöchern (116), wobei der Aluminiumboden (104) an dem Aluminiumverschluss (102,226) befestigbar ist;
einem ersten und zweiten Filtermedium (124,206,212), welches für den Gasplasmafluss durchlässig ist, die mit dem ersten und dem zweiten Satz von Lüftungslöchern (114,116;202,204) entsprechend in Zusammenhang stehen.
**dadurch gekennzeichnet, dass**
eine Eloxalschicht im Wesentlichen auf den Aluminiumverschluss (102,226) und den Alumniniumboden (104) aufgebracht ist, wobei die Eloxalschicht eine Dicke aufweist, die im Wesentlichen 0,5 mils (0,0005 Inches; 12,7 µm) nicht überschreitet.

2. Metallischer Sterilisationsbehälter nach Anspruch 1, wobei das Aluminium 6061 T6 umfasst.

3. Metallischer Sterilisationsbehälter nach Anspruch 1, wobei die Eloxalschicht eine Dicke aufweist, die im Wesentlichen 0,3 mils (0,0003 Inches; 7,62 µm) nicht überschreitet.

4. Metallischer Sterilisationsbehälter nach Anspruch 1, wobei die Eloxalschicht eine Dicke aufweist, die im Wesentlichen unter 0,2 mils (0,0002 Inches; 5,08 µm) liegt.

5. Metallischer Sterilisationsbehälter nach Anspruch 1, wobei die Eloxalschicht eine Dicke aufweist, die im Wesentlichen zwischen 0,2 mils (0,0002 Inches; 5,08 µm) und 0,3 mils (0,0003 Inches; 7,62 µm) liegt.

6. Metallischer Sterilisationsbehälter nach Anspruch 1, wobei die Eloxalschicht eine Dicke aufweist, die im Wesentlichen zwischen 0,2 mils (0,0002 Inches; 5,08 µm) und 0,5 mils (0,0005 Inches; 12,7 µm) liegt.

7. Metallischer Sterilisationsbehälter nach Anspruch 1, wobei die Eloxalschicht eine Dicke aufweist, die im Wesentlichen zwischen 0,3 mils (0,0003 Inches; 7,62 µm) und 0,5 mils (0,0005 Inches; 12,7 µm) liegt.

8. Metallischer Sterilisationsbehälter nach Anspruch 1, wobei der erste Satz von Lüftungslöchern und der zweite Satz von Lüftungslöchern (114,116;202,204) gegeneinander versetzt angeordnet sind.

9. Metallischer Sterilisationsbehälter nach Anspruch 1, welcher ferner eine Dichtung aufweist, die zwischen dem Aluminiumverschluss und dem Aluminiumboden befestigbar ist.

10. Metallischer Sterilisationsbehälter nach Anspruch 1, welcher ferner eine Filterrückhalteplatte (32) umfasst, welche an dem Aluminiumverschluss (16) befestigbar ist, wobei die Filterrückhalteplatte (32) einen Satz von Lüftungslöchern aufweist, die im Wesentlichen relativ zu dem ersten Satz von Lüftungslöchern (114) versetzt angeordnet ist.

11. Metallischer Sterilisationsbehälter nach Anspruch 1, wobei die erste Rückhalteplatte (32,128) und der Aluminiumverschluss (16,102) einen Abstand in dem Bereich der Lüftungslöcher (114) bilden, in welchem das erste Filtermedium (20,124) positioniert ist.

12. Metallischer Sterilisationsbehälter nach Anspruch 1, wobei der Aluminiumboden (108) und die zweite Rückhalteplatte (154) einen Abstand in dem Bereich des zweiten Satzes von Lüftungslöchern bilden, in welchem das zweite Filtermedium positioniert ist.

13. Metallischer Sterilisationsbehälter nach Anspruch 1, welcher ferner eine Dichtung (46,48) umfasst, die an der ersten Rückhalteplatte (32) befestigt ist, und welche so positioniert ist, dass sie in einer Dichtungsposition mit dem Verschluss eine Abdichtung erzeugt.

14. Metallischer Sterilisationsbehälter nach Anspruch 1, welcher eine Dichtung oder mehrere Dichtungen (46,48) umfasst.

15. Metallischer Sterilisationsbehälter nach Anspruch 13, wobei die Dichtung (46,48) aus Silikon, Neopren oder Teflon® hergestellt sein kann.

16. Metallischer Sterilisationsbehälter nach Anspruch 1, welcher ferner eine Dichtung umfasst, die an der zweiten Rückhalteplatte positioniert ist, um in einer Dichtungsposition eine Abdichtung mit dem Boden zu erzeugen.

17. Metallischer Sterilisationsbehälter nach Anspruch 1, wobei das Filtermedium aus Papier, Teflon, porösem rostfreiem Stahl, Polysulfon, hydrophobem Material und Mischungen daraus hergestellt ist.

18. Metallischer Sterilisationsbehälter nach Anspruch 1, welcher ferner einen dritten Satz von Lüftungslöchern umfasst, die im Wesentlichen in dem Aluminiumboden angeordnet sind.

19. Metallischer Sterilisationsbehälter nach Anspruch 1, wobei der Aluminiumverschluss und der Alumniumboden im Wesentlichen gegeneinander elektrisch isoliert sind.

20. System für die Sterilisation von Gegenständen in einem Behälter mit einem Gasplasma, wobei das System umfasst:
Mittel zum Einführen von Gasplasma und den Behälter nach Anspruch 1.

21. Verfahren für die Sterilisation von Instrumenten in einem metallischen Behälter mit einem Verschluss und einem Boden, wobei der Verschluss einen ersten Satz von Lüftungslöchern und ein erstes Filtermedium, der Boden einen zweiten Satz von Lüftungslöchern und ein zweites Filtermedium aufweist, und das Verfahren die folgenden Schritte umfasst:
Positionieren von mindestens einem Instrument in dem metallischen Behälter;
Durchströmen des ersten Satzes von Lüftungslöchern und des ersten Filtermediums mit einem Gasplasmasterilisations- medium;
Hervorrufen eines turbulenten Durchstroms des Gasplasmasterilisationsmediums innerhalb des metallischen Behälters;
Im Wesentlichen beschränken des elektrischen Stroms zwischen dem Verschluss und dem Boden, wobei der Verschluss und der Boden eine Anodisierungsschicht umfassen mit einer Dicke, die nicht größer ist als 0,5 mils (0,0005 Inches; 12,7 µm); und
Durchströmen des zweiten Satzes von Lüftungslöchern und des zweiten Filtermediums mit dem Gasplasmasterilisationsmedium.

22. Verfahren für die Herstellung eines Aluminiumsterilisationsbehälters mit einem Verschluss und einem Boden, wobei das Verfahren die Schritte umfasst:
Bilden eines ersten Satzes von Lüftungslöchern in dem Verschluss:
Bilden eines zweiten Satzes von Lüftungslöchern in dem Boden; und
Auftragen einer im Wesentlichen einheitlichen Eloxalschicht auf den Verschluss und den Boden, wobei die
Eloxalschicht eine Dicke aufweist, die nicht größer ist als 0,5 mils (0,0005 Inches; 12,7 µm).

23. Metallischer Sterilisationsbehälter nach Anspruch 1 welcher ferner eine zweite Filterrückhalteplatte umfasst, die an dem Aluminiumboden befestigbar ist, wobei die Filterrückhalteplatte einen Satz von Lüftungslöchern aufweist, welche im Wesentlichen relativ zum zweiten Satz von Lüftungslöchern versetzt angeordnet ist.

24. Metallischer Sterilisationsbehälter nach Anspruch 16, wobei es sich bei dem hydrophoben Material um Polyethylen/Polypropylen handelt.

## Revendications

1. Récipient de stérilisation métallique (100, 200) destiné à stériliser des objets dans un support de stérilisation à plasma gazeux, le récipient (100, 200) comprenant :
un couvercle en aluminium (102, 226) ayant un premier ensemble de trous d'aération (114, 202 ; 204) ;
une partie inférieure en aluminium (104) ayant un deuxième ensemble de trous d'aération (116), la partie inférieure en aluminium (104) pouvant être attachée au couvercle en aluminium (102, 226) ;
un premier et un second éléments filtrants (124, 206, 212) perméables au flux du plasma gazeux, respectivement associés au premier et au second ensembles de trous d'aération (114, 116 ; 202, 204)
**caractérisé en ce que**
un revêtement anodique est appliqué au couvercle en aluminium (102, 226) et à la partie inférieure en aluminium (104), le revêtement anodique ayant une épaisseur qui n'est pas sensiblement supérieure à 0,5 mils (0,0005 pouces ; 12,7 µm)

2. Récipient de stérilisation métallique selon la revendication 1, dans lequel l'aluminium comprend du 6061 T6.

3. Récipient de stérilisation métallique selon la revendication 1, dans lequel le revêtement anodique comprend une épaisseur qui n'est pas sensiblement supérieure à 0,3 mils (0,0003 pouces, 7,62 µm).

4. Récipient de stérilisation métallique selon la revendication 1, dans lequel le revêtement anodique comprend une épaisseur sensiblement inférieure à 0,2 mils (0,0002 pouces ; 5,08 µm).

5. Récipient de stérilisation métallique selon la revendication 1, dans lequel le revêtement anodique comprend une épaisseur comprise sensiblement entre 0,2 mils (0,0002 pouces ; 5,08 µm) et 0,3 mils (0,0003 pouces ; 7,62 µm).

6. Récipient de stérilisation métallique selon la revendication 1, dans lequel le revêtement anodique comprend une épaisseur comprise sensiblement entre 0,2 mils (0,0002 pouces ; 5,08 µm) et 0,5 mils (0,0005 pouces ; 12,7 µm).

7. Récipient de stérilisation métallique selon la revendication 1, dans lequel le revêtement anodique comprend une épaisseur comprise sensiblement entre 0,3 mils (0,0003 pouces ; 7,62 µm) et 0,5 mils (0,0005 pouces ; 12,7 µm).

8. Récipient de stérilisation métallique selon la revendication 1, dans lequel le premier ensemble de trous d'aération et le second ensemble de trous d'aération (114, 116 ; 202, 204) sont décalés l'un par rapport à l'autre.

9. Récipient de stérilisation métallique selon la revendication 1, comprenant en outre un joint pouvant être attaché entre le couvercle en aluminium et la partie inférieure en aluminium.

10. Récipient de stérilisation métallique selon la revendication 1, comprenant en outre une plaque de retenue de filtre (32) pouvant être attachée au couvercle en aluminium (16), la plaque de retenue de filtre (32) ayant un ensemble de trous d'aération sensiblement décalé par rapport au premier ensemble de trous d'aération (114).

11. Récipient de stérilisation métallique selon la revendication 1, dans lequel la première plaque de retenue (32, 128) et le couvercle en aluminium (16, 102) définissent un espace dans la région des trous d'aération (114) dans lequel est placé le premier élément filtrant (20, 124).

12. Récipient de stérilisation métallique selon la revendication 1, dans lequel la partie inférieure en aluminium (108) et la seconde plaque de retenue (154) définissent un espace dans la région du deuxième ensemble de trous d'aération dans lequel est placé le second élément filtrant.

13. Récipient de stérilisation métallique selon la revendication 1, comprenant en outre un joint (46, 48) attaché à ladite première plaque de retenue (32) et positionné de façon à créer une étanchéité avec ledit couvercle quand il est dans une position d'étanchéité.

14. Récipient de stérilisation métallique selon la revendication 1, comprenant un ou plusieurs joints (46, 48).

15. Récipient de stérilisation métallique selon la revendication 13, dans lequel le joint (46, 48) peut être réalisé en silicone, en néoprène ou en Teflon®.

16. Récipient de stérilisation métallique selon la revendication 1, comprenant en outre un joint attaché à ladite seconde plaque de retenue et positionné de façon à créer une étanchéité avec ladite partie inférieure quand il est dans une position d'étanchéité.

17. Récipient de stérilisation métallique selon la revendication 1, dans lequel l'élément filtrant est réalisé en papier, en Teflon, en acier inoxydable poreux, en polysulfone, en matériau hydrophobe et en mélanges de ceux-ci.

18. Récipient de stérilisation métallique selon la revendication 1, comprenant en outre un troisième ensemble de trous d'aération sensiblement situé dans la partie inférieure en aluminium.

19. Récipient de stérilisation métallique selon la revendication 1, dans lequel le couvercle en aluminium et la partie inférieure en aluminium sont sensiblement électriquement isolés l'un de l'autre.

20. Système de stérilisation d'objets dans un récipient avec un plasma gazeux, le système comprenant :
des moyens pour introduire un plasma gazeux et le récipient selon la revendication 1.

21. Procédé de stérilisation d'instruments dans un récipient métallique comprenant un couvercle et une partie inférieure, le couvercle ayant un premier ensemble de trous d'aération et un premier élément filtrant, la partie inférieure ayant un deuxième ensemble de trous d'aération et un second élément filtrant, le procédé comprenant les étapes consistant à:
placer au moins un instrument dans le récipient métallique ;
faire passer un milieu de stérilisation au plasma gazeux à travers le premier ensemble de trous d'aération et le premier élément filtrant ;
provoquer l'écoulement turbulent le milieu de stérilisation au plasma gazeux dans le récipient métallique ;
limiter sensiblement le courant électrique entre le couvercle et la partie inférieure, dans lequel le couvercle et la partie inférieure comprennent une couche d'anodisation avec une épaisseur qui n'est pas supérieure à 0,5 mils (0,0005 pouces ; 12,7 µm) ; et
faire passer le milieu de stérilisation au plasma gazeux à travers le deuxième ensemble de trous d'aération et le second élément filtrant.

22. Procédé de fabrication d'un récipient de stérilisation métallique ayant un couvercle et une partie inférieure, le procédé comprenant les étapes consistant à :
former un premier ensemble de trous d'aération dans le couvercle ;
former un deuxième ensemble de trous d'aération dans la partie inférieure ; et
appliquer un revêtement anodique sensiblement uniforme au couvercle et à la partie inférieure, le revêtement anodique ayant une épaisseur qui n'est pas supérieure à 0,5 mils (0,0005 pouces ; 12,7 µm).

23. Récipient de stérilisation métallique selon la revendication 1, comprenant en outre une seconde plaque de retenue de filtre pouvant être attachée à la partie inférieure en aluminium, la plaque de retenue de filtre ayant un ensemble de trous d'aération sensiblement décalé par rapport au deuxième ensemble de trous d'aération.

24. Récipient de stérilisation métallique selon la revendication 16, dans lequel ledit matériau hydrophobe est un polyéthylène/polypropylène.
